# EUROPEAN PATENT SPECIFICATION

(11) **EP 1 319 656 B1**
(45) Date of publication and mention of the grant of the patent: **23.08.2006**
(21) Application number: 01980134.9
(22) Date of filing: 06.07.2001
(51) Int. Cl.: C07D 215/56, C07C 49/78, C07C 49/76, C07C 49/807

(54) **A NEW PROCESS FOR PREPARING A QUINOLONE-CARBOXYLIC ACID**
NEUES VERFAHREN ZUR HERSTELLUNG EINER CHINOLON-CARBONSÄURE
NOUVEAU PROCEDE DE PREPARATION D'UN ACIDE QUINOLONE-CARBOXYLIQUE

(30) Priority: 16.08.2000 CN 00123446
(43) Date of publication of application: 18.06.2003
(73) Proprietor: Lynchem Co., Ltd., Dalian, Liaoning 116105 (CN)
(72) Inventor: WANG, Yuncai, Dalian, Liaoning Province 116001 (CN); CHEN, Rongye, Dalian, Liaoning Province 116001 (CN); NAN, Haijun, Dalian, Liaoning Province 116001 (CN)
(74) Representative: Harrison Goddard Foote
(86) International application number: PCT/CN2001/001156
(87) International publication number: WO 2002/059094

(56) References cited:
- ES-A- 2 049 640
- US-A- 4 730 000

## Description

### Field of the Invention

The present invention relates to a process for preparing quinolinecarboxylic acids, particularly to a process for preparing quinolinecarboxylic acids from acetylbenzene polyhalides.

### Background of the Invention

People have increasingly paid attention to anti-infection drugs of quinolines due to their excellent properties. Quinolinecarboxylic acids are key intermediates of the anti-infection drugs since all the drugs of quinolines are based on such intermediates followed by a series of reactions. Therefore, the synthesis of the intermediates of quinolinecarboxylic acids is very important in the preparation of the quinoline drugs.

Various methods of preparing qui quinolinecarboxylic acids have been disclosed in the prior art, see for example US 4,730,000 (D1) and ES 2 049640 (D2). For example, Gaticarboxylic acid is prepared starting from 2,4,5-trifluoro-3-methoxylbenzene carboxylic acid, and Sparcarboxylic acid is prepared starting from pentafluorobenzenecarboxylic acid. However, the yield of the subject compound in these methods is not satisfied. The invention is hereby provided to overcome the drawbacks.

### Summary of the Invention

One object of the invention is to provide a new process for preparing quinolinecarboxylic acid derivatives starting from acetylbenzene polyhalides. The process according to the invention has fewer steps but a higher yield to obtain the subject compound.

According to the present invention, the process for preparing quinolinecarboxylic acid derivatives having the formula (I): 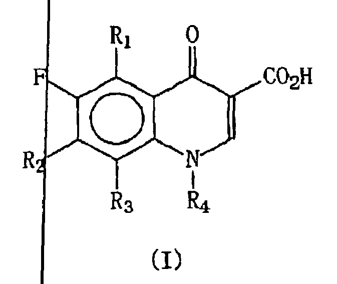 wherein R₁ is H, halogen, or amino,
R₂ is halogen,
R₃ is halogen, C₁₋₄alkoxyl, or CN,
R₄ is selected from the group consisting of C₃₋₆cycloalkyl, C₁₋₄alkyl, C₁₋₄alkoxylC₁₋₄alkyl, and C₁₋₄alkylaminoC₁₋₄alkyl,
comprising the steps of:
i) reacting a compound of the formula (II) 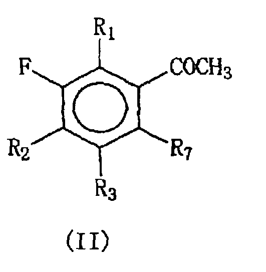 wherein R₁, R₂ and R₃ are defined as above, and R₇ is a halogen, with an alkyl carbonate to obtain a compound of the formula (III), 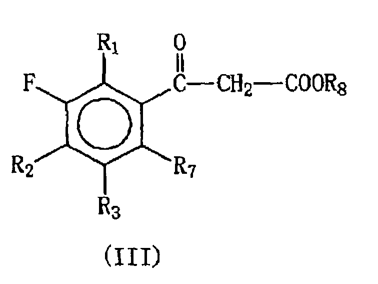 wherein R₈ is methyl or ethyl;
ii) reacting a compound of the formula (III) successively with an alkyl orthoformate and a compound of the formula (IV) R₄NH₂ to obtain a compound of the formula (V), 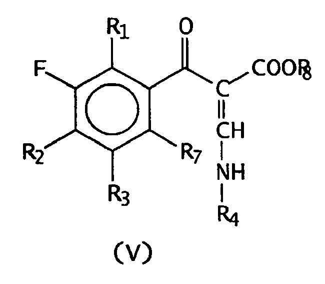
iii) cyclizing the compound of the formula (V) in the presence of a base to form a compound of the formula (VI); and 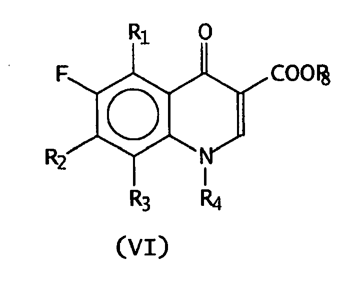
iv) hydrolyzing the compound of the formula (VI) to obtain a compound of the formula (I).

The another object of the invention is to provide some compounds having the formula (II),

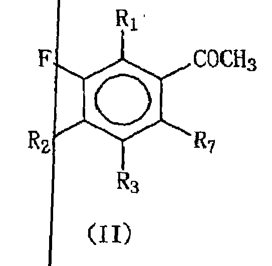

In the formula (II), those compounds, in which R₁ is H, or Cl; R₂ is Cl; R₃ is C₁₋₄ alkoxy or CN; and R₇ is Cl, are novel compounds.

### Detailed Description of the Invention

In the process for preparing compounds of the formula (I) according to the present invention, R₁ is preferably H, F, or NH₂; R₂ is preferably F or Cl; R₃ is preferably F, CN or CH₃; and R₄ is preferably cyclopropyl or cyclohexyl.

Methyl carbonate or ethyl carbonate can be used in the reaction with compounds having the formula (II). This reaction in general is carried out in the presence of a base in organic solvents. Preferably, carbonates themselves are used as reaction solvents. The base used herein may be NaH or sodium alkoxide.

In general, the reaction between compounds of the formula (III) and orthoformates may be carried out in the presence of diacetyl oxide, and the resultant may be directly reacted with R₄NH₂ of the formula (IV) to obtain a compound having the formula (V).

Cyclization of the compound of the formula (V) can be carried out in an organic solvent in the presence of a base to obtain a compound having the formula (VI).

Preferable solvent is DMF, and anh ous sodium carbonate may be selected as the base.

The compound of the formula (VI) can be hydrolyzed to give the corresponding compound of the formula (I) using a conventional method in the art.

In the process of the present invention, the compound of the formula (I) in which R₁ is NH₂ can be converted from a compound thereof in which R₁ is halogen. The reaction scheme is shown as follows:

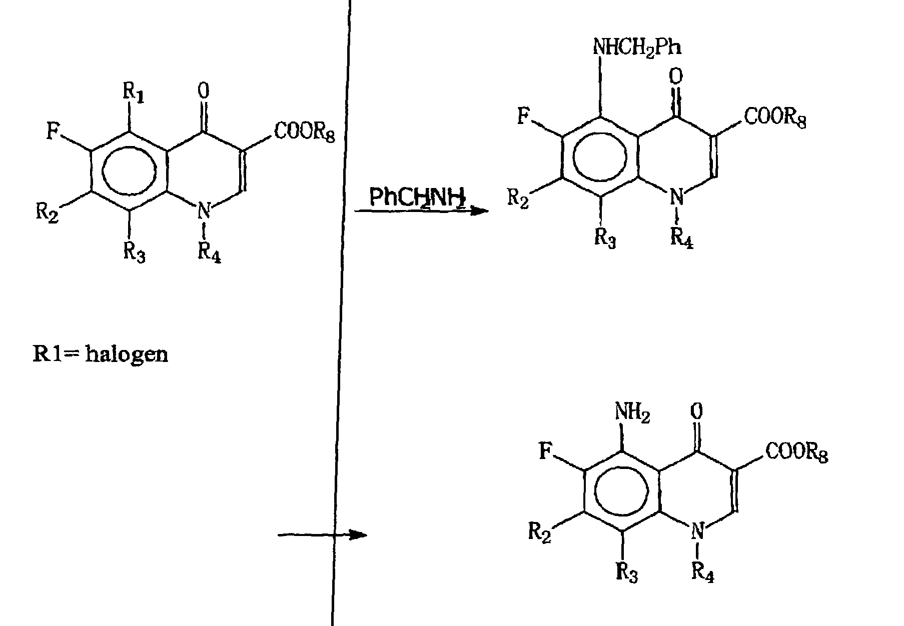

In the compound of the formula (II), R₁ is preferably H, and R₃ is preferably OCH3 or CN.

Although the compound having the formula (II) can be prepared by many methods in the art, it is prepared in the invention starting from benzene polyhalides that can be easily obtained commercially.

The invention will be further illustrated with the following examples.

### Preparation of Quinolinecarboxylic Acids

### Example 1

### Gaticarboxylic acids prepared from 2,4-dichloro-5-fluoro-3-methoxyacetylbenzene

### Methyl 2,4-dichloro-5-fluoro-3-methoxybenzoylacetate

80.6g of 2,4-dichloro-5-fluoro-3-methoxyacetylbenzene (0.34mol) in 1000ml of dimethyl carbonate was stirred. 50g of 50% NaH (1.04mol) was added in batch to the resulting solution at ambient temperature. After the addition, the reaction was carried out at 80°C for 3 hours. The reactant was then poured into ice water containing a little amount of acetate acid. The resulting mixture was extracted with ethyl ether. The extract was washed with water, dried over anhydrous sodium sulfate. The residue was recrystallized from methanol after ethyl ether was evaporated and excessive dimethyl carbonate was recovered from the solution, thereby to afford 80.8g of methyl 2,4-dichloro-5-fluoro-3-methoxybenzoylacetate (0.274mol) in 80.6% yield having a melt point of 50-53°C.

### Methyl 2-(2,4-dichloro-5-fluoro-3-methoxybenzoyl)-3-cyclopropylaminoacrylate

73.8g of methyl 2,4-dichloro-5-fluoro-3-methoxybenzoylacetate (0.25mol), 66.6g of triethyl orthoformate (0.45mol) and 77.4g of diacetyl oxide (0.72mol) were stirred at 150°C for 2.5 hours. Fractions having a lower boiling point were evaporated under reduced pressure. To the residue was added 250ml of anhydrous ethanol. 14.5g of cyclopropylamine (0.25mol) was added to the solution and the reaction was then carried out for 2 hours. The resulting mixture was filtered under suction. The residue was re-crystallized from a mixture of petroleum ether and cyclohexane to yield 66.0g (0.182mol) of methyl 2-(2,4-dichloro-5-fluoro-3-methoxybenzoyl)-3-cyclopropylaminoacrylate in 72.9% yield.

### Methyl 1-cyclopropyl-7-chloro-6-fluoro-8-methoxy-1,4-dihydro-4-oxo-3-quinoline-carboxylate

34.7g of anhydrous K₂CO₃ (0.35mol) was added to a solution of 62.6g of methyl 2-(2,4-dichloro-5-fluoro-3-methoxybenzoyl)-3-cyclopropylaminoacrylate in 220ml of DMF. The reactant was stirred at 40-45°C for 2.5 hours, and the reaction was monitored by TLC. The resulting mixture was then poured into 800ml of ice-water, filtered, washed with water (100ml X 2), and dried. The resulting solid was dissolved in 120ml of 95% methanol and then refluxed for 15 minutes, cooled, filtered and dried to afford 50.8g of methyl 1-cyclopropyl-7-chloro-6-fluoro-8-methoxy-1,4-dihydro-4-oxo-3-quinolinecarboxylate (0.156mol) as a white solid, mp 184-187C, in 90.1% yield.

### 1-Cyclopropyl-7-chloro-6-fluoro-8-methoxy-1,4-dihydro-4-oxo-3-quinolinecarboxylic acid

28.6g of methyl 1-cyclopropyl-7-chloro-6-fluoro-8-methoxy-1,4-dihydro-4-oxo-3-quinolinecarboxylate (0.088mol), 160ml of acetate acid, 100ml of water and 18ml of concentrated sulfuric acid were stirred for 40 minutes at 100-110°C. The resulting mixture was cooled and filtered. The precipitate was re-crystallized from choroform-ethanol to afford 25.2g of 1-cyclopropyl-7-chloro-6- fluoro-8-methoxy-1,4-dihydro-4-oxo-3-quinolinecarboxylic acid (Gaticarboxylic acid, 0.081mol) in 91.8% yield, mp 195-199°C.

### Example 2

### 8-cyano-quinolinecarboxylic acid from 3-cyano-2,4-dichloro-5-fluoroacetylbenzene

### Ethyl 3-cyano-2,4-dichloro-5-fluorobenzyolacetate

78.9g of 3-cyano-2,4-dichloro-5-fluoroacetylbenzene (0.34mol) in 1000ml of diethyl carbonate was stirred. To the solution was added in batch 50.0g of 50% NaH (1.04mol) at room temperature. The reaction was carried out at 80°C for 3 hours. The resultant was carefully poured into ice-water containing a little mount of acetate acid, extracted with ethyl ether. The extract was washed with water, dried over anhydrous sodium sulfate. The residue was recrystallized from toluene after ethyl ether was evaporated and excessive dimethyl carbonate was recovered from the solution to afford 86.6g of ethyl 3-cyano-2,4-dichloro-5-fluorobenzoylacetate (0.285mol) in 83.7% yield.

### Ethyl 2-(3-cyano-2,4-dichloro-5-fluorobenzoyl)-3-cyclopropylaminoacrylate

76.0g of ethyl 3-cyano-2,4-dichloro-5-fluorobenzoylacetate (0,25mol), 66.6g of triethyl orthoformate (0.45mol) and 77.4g of diacetyl oxide (0.72mol) were stirred at 150°C for 2.5 hours. Fractions having a lower boiling point were evaporated under reduced pressure. To the residue was added 250ml of anhydrous ethanol. 14.5g of cyclopropylamine (0.25mol) was added to the solution and the reaction was then carried out for 2 hours. The resulting mixture was filtered under suction. The residue was recrystallized from a mixture of petroleum ether and cyclohexane to yield 65.0g (0.175mol) of ethyl 2-(3-cyano-2,4-dichloro-5-fluorobenzoyl)-3-cyclopropylamino-acrylate in 70.1% yield.

### Ethyl 8-cyano-1-cyclopropyl-7-chloro-6-fluoro-1,4-dihydro-4-oxo-3-quinoline-carboxylate

34.7g of anhydrous K₂CO₃ (0.35mol) was added to a solution of 64.2g of ethyl 2-(3-cyano-2,4-dichloro-5-fluorobenzoyl)-3-cyclopropylaminoacrylate (0.173mol) in 220ml of DMF. The reacting mixture was stirred at 40-45°C for 2.5 hours, and the reaction was monitored by TLC. The reactant was then poured into 800ml of ice-water, filtered, washed with water (100ml X 2), and dried. The resultant solid was dissolved in 120ml of 95% ethanol and then refluxed for 15 minutes, cooled, filtered and dried to afford 53.2g of ethyl 8-cyano-1-cyclopropyl-7-chloro-6-fluoro-1,4-dihydro-4-oxo-3-quinolinecarboxylate
(0.159mol) as a white solid, mp 196-199°C, in 90.1% yield.

### 8-cyano-1-cyclopropyl-7-chloro-6-fluoro-1,4-dihydro-4-oxo-3-quinolinecarboxylic acid

29.4g of ethyl 1-cyclopropyl-7-chloro-6-fluoro-8-methoxy-1,4-dihydro-4-oxo-3-quinolinecarboxylate (0.088mol), 160ml of acetate acid, 100ml of water and 18ml of concentrated sulfuric acid were stirred at 100-110°C for 40 minutes. The resulting mixture was cooled and filtered. The precipitate was re-crystallized from chloroform-ethanol to give 23.8g of 1-cyclopropyl-7-chloro-6- fluoro-8-methoxy-1,4-dihydro-4-oxo-3-quinolinecarboxylic acid (0.0775mol) in 88.1% yield, mp 288-293°C.

### Example 3

### Sparcarboxylic acids prepared from pentafluoroacetylbenzene

### Ethyl pentafluorobenzoylacetate

71.4g (0.34mol) of pentafluoroacetylbenzene in 1000ml of diethyl carbonate was stirred. 50.0g (1.04mol) of 50% NaH was added in batch to the resulting solution at ambient temperature. After the addition, the reaction was carried out at 80°C for three hours. The reactant was then poured into ice-water containing a little amount of acetate acid. The resulting mixture was extracted with ethyl ether. The extract was washed with water, dried over anhydrous sodium sulfate. The residue of the solution was re-crystallized from methanol after ethyl ether was evaporated and excessive dimethyl carbonate was recovered from the solution. 80.5g of ethyl pentafluorobenzoylacetate (112-121°C/8Pa, 0.286mol) was given under reduced pressure in 80.6% yield.

### Ethyl 2-(pentafluorobenzoyl) -3-cyclopropylaminoacrylate

70.5g of ethyl pentafluorobenzoylacetate (0.25mol), 66.6g of triethyl orthoformate (0.45mol) and 77.4g of diacetyl oxide (0.72mol) were stirred at 150°C for 2.5 hours. Fractions having a lower boiling point were evaporated under reduced pressure. To the residue was added 250ml of anhydrous ethanol. 14.5g of cyclopropylamine (0.25mol) was added to the solution under cooling of ice water and the reaction was then carried out at room temperature for 2 hours. The resulting mixture was filtered under suction. The residue was re-crystallized from a mixture of petroleum ether and cyclohexane to yield 62.8g (0.18mol) of ethyl 2-(pentafluorobenzoyl)-3-cyclopropylaminoacrylate in 72.0% yield, mp 88-91°C.

### Ethyl 1-cyclopropyl-5,6,7,8-tetrafluoro-1,4-dihydro-4-oxo-3-quinolinecarboxylate

34.7g of anhydrous K₂CO₃ was added to a solution of 60.4g of ethyl 2-pentafluorobenzoyl-3-cyclopropylaminoacrylate (0.173mol) in 220ml of DMF. The reactants were stirred at 40-45°C for 2.5 hours, and the reaction was monitored by TLC. The resulting mixture was then poured into 800ml of ice-water, filtered, washed with water (100ml X 2), and dried. The resulting solid was dissolved in 120ml of 95% methanol and then refluxed for 15 minutes, cooled, filtered and dried to give 53.0g of ethyl 1-cyclopropyl-5,6,7,8-tetrafluoro-1,4-dihydro-4-oxo-3-quinolinecarboxylate (0.156mol) as a white solid, mp 169-171°C, in 93% yield.

### Ethyl 5-benzylamino-1-cyclopropyl-6,7,8-trifluoro-1,4-dihydro-4-oxo-3-quinoline-carboxylate

53.0g of ethyl 1-cyclopropyl-5,6,7,8-tetrafluoro-1,4-dihydro-4-oxo-3-quinoline-carboxylate (0161mol), 15.4ml of benzyl amine, and 37.0g of anhydrous K₂CO₃ in 220ml of acetonitrile were stirred at 100-110°C for 1 hour. The reaction was monitored by TLC. After the solvent was evaporated, the residue was re-crystallized from ethanol to give 53.7g of ethyl 1-cyclopropyl-6,7,8-trifluoro-1,4-dihydro-4- oxo-3-quinolinecarboxylate (0.129mol) in 80.0% yield, mp 133-135°C.

### Ethyl 5-amino-1-cyclopropyl-6,7,8-trifluoro-1,4-dihydro-4-oxo-3-quinolinecarboxylate

To a solution of 46.2g of ethyl 5-benzylamino-1-cyclopropyl-6,7,8-trifluoro-1,4-dihydro-4-oxo-3-quinolinecarboxylate (0.111mol) dissolved in a mixture of 200ml of acetic acid and ethanol were added 0.5g of 5% Pd/C catalyst. Hydrogen gas was introduced at room temperature and monitored with TLC. The reaction was carried out for around 3 hours. Precipitate collected by filtration was dissolved in chloroform and filtered to remove the catalyst. After removing the solvent by evaporation, the residue was crystallized from a mixture of chloroform and ethanol to give 32.4g of ethyl 5-amino-1-cyclopropyl-6,7,8-trifluoro-1,4-dihydro-4-oxo-3-quinolinecarboxylate (0.0944mol) in 85.0% yield, mp. 235-237°C.

### Sparcarboxylic acid: 5-amino-1-cyclopropyl-6,7,8-trifluoro-1,4-dihydro-4-oxo-3-quinolinecarboxylic acid

28.7g of ethyl 5-amino-1-cyclopropyl-6,7,8-trifluoro-1,4-dihydro-4-oxo-3-quinoline-carboxylate (0.088mol), 160ml of acetic acid, and a mixture of 100ml of water and 18ml of concentrated sulfuric acid were stirred at 100-110°C for 40 minutes. The reacting mixture was cooled and filtered. The precipitate was recrystallized from chloroform-ethanol to give 25.6g 5-amino-1-cyclopropyl-6,7,8-trifluoro-1,4-dihydro-4-oxo-3-quinolinecarboxylic acid (Sparcarboxylic acid, 0.086mol), mp. 293-295C, in 98.0% yield.

### Example 4

### Sparcarboxylic acids prepared from 2,4,6-trichloro-3,5-difluoroacetylbenzene

### Ethyl 2,4,6-trichloro-3,5-difluorobenzoylacetate

88.2g of 2,4,6-trichloro-3,5-difluoroacetylbenzene(0.34mol) in 1000ml of diethyl carbonate was stirred. 50.0g (1.04mol) of 50% NaH was added in batch to the resulting solution at ambient temperature. After the addition, the reaction was carried out at 80°C for 3 hours. The reactant was then poured into ice-water containing a little amount of acetate acid. The resulting mixture was extracted with ethyl ether. The extract was washed with water, dried over anhydrous sodium sulfate. The residue of the solution was re-crystallized from toluene, after ethyl ether was evaporated and excessive diethyl carbonate was recovered from the solution to give 93.3g of ethyl 2,4,6-trichloro-3,5-fluorobenzoylacetate (0.276mol) in 81.2% yield.

### Ethyl 2-(2,4,6-trichloro-3,5-difluorobenzoyl)-3-cyclopropylaminoacrylate

82.9g of ethyl 2,4,6-trichloro-3,5-difluorobenzoylacetate (0.25mol), 66.6g of triethyl orthoformate (0.45mol) and 77.4g of diacetyl oxide (0.72mol) were stirred at 150°C for 2.5 hours. Fractions having a lower boiling point were evaporated under reduced pressure. To the residue was added 250ml of anhydrous ethanol. 14.5g of cyclopropylamine (0.25mol) was added to the solution under cooling of ice water and the reaction was then carried at room temperature for 2 hours. The resultant mixture was filtered under suction. The residue was re-crystallized from a mixture of petroleum ether and cyclohexane to yield 74.5g (0.187mol) of ethyl 2-(2,4,6-trichloro-3,5-difluorobenzoyl)-3-cyclopropylaminoacrylate in 74.6% yield.

### Ethyl 1-cyclopropyl-5,7-dichloro-6,8-difluoro-1,4-dihydro-4-oxo-3-quinolinecarboxylate

34.7.0g of anhydrous K₂CO₃ was added to a solution of 68.9g of ethyl 2-(2,4,6-trichloro-3,5-difluorobenzoyl-3-cyclopropylaminoacrylate (0.173mol) in 220ml of DMF. The reacting mixture was stirred at 40-45°C for 2.5 hours, and the reaction was monitored by TLC. The resultant was then poured into 800ml of ice-water. The resulting mixture was filtered. The precipitate was washed with water (100ml X 2), and dried. The resulting solid was dissolved in 120ml of 95% ethanol and then refluxed for 15 minutes. The resulting mixture was cooled, filtered and dried to give 60.4g of ethyl 1-cyclopropyl-5,6,7,8-tetrafluoro-1,4-dihydro-4-oxo-3-quinolinecarboxylate (0.167mol) as a white solid, mp 191-195°C, in 96.4% yield.

### Ethyl 5-benzylamino-1-cyclopropyl-7-chloro-6,8-difluoro-1,4-dihydro-4-oxo-3-quinolinecarboxylate

58.3g of ethyl 1-cyclopropyl-5,7-dichloro-6,8-difluoro-1,4-dihydro-4-oxo-3-quinoline carboxylate (0161mol), 15.4ml of benzyl amine, and 37.0g of anhydrous K₂CO₃ in 220ml of acetonitrile were stirred at 100-110°C for 1 hour. The reaction was monitored by TLC. After the solvent was evaporated, the residue was re-crystallized from ethanol to give 58.3g of ethyl 1-cyclopropyl-7-chloro -6,8-difluoro-1,4-dihydro-4-oxo-3-quinolinecarboxylate (0.135mol) in 83.7% yield.

### Ethyl 5-amino-1-cyclopropyl-7-chloro-6,8-difluoro-1,4-dihydro-4-oxo-3-quinolinecarboxylate

To a solution of 48.0g of ethyl 5-benzylamino-1-cyclopropyl-7-chloro-6,8- difluoro--1,4-dihydro-4-oxo-3-quinolinecarboxylate (0.111mol) dissolved in a mixture of 200ml of acetic acid and ethanol was added 0.5g of 5% Pd/C catalyst. Hydrogen gas was introduced at room temperature. The reaction was monitored with TLC and carried out for around 3 hours. Precipitates collected by filtration were dissolved in chloroform and filtered to remove the catalyst. After removing the solvent by evaporation, the residue was recrystallized from a mixture of chloroform and ethanol to give 32.0g of ethyl 5-amino-1-cyclopropyl-7-chloro-6,8-difluoro-1,4-dihydro-4-oxo-3-quinolinecarboxylate (0.093mol) in 83.9% yield.

### Sparcarboxylic acid: 5-amino-1-cyclopropyl-7-chloro-6,8-difluoro-1,4-dihydro-4-oxo-3-quinolinecarboxylic acid

30.1g of ethyl 5-amino-1-cyclopropyl-6,7,8-trifluoro-1,4-dihydro-4-oxo-3-quinoline-carboxylate (0.088mol), 160ml of acetic acid, and a mixture of 100ml of water and 18ml of concentrated sulfuric acid were stirred at 100-110°C for 40 minutes. The reacting mixture was cooled and filtered. The precipitate was crystallized from chloroform-ethanol to give 26.8g of 5-amino-1-cyclopropyl-7-chloro-6,8-difluoro-1,4-dihydro-4-oxo-3-quinolinecarboxylic acid (Sparcarboxylic acid, 0.0852mol), mp. 283-287C, in 96.8% yield.

### Preparation of halogen acetylbenzene

### Example 5 Preparation of 2,4-dichloro-5-fluoro-3-methoxyacetylbenzene

### a. Preparation of 2,4-dichloro-5-fluoronitrobenzene

58.3ml of 2,4-dichrolofluorobenzene (0.50mol) was added to a mixture of 60ml of concentrated nitric acid (1.41mol), 93ml of concentrated sulfuric acid (1.75mol) and 16.5ml of water. The reaction was carried out at 50-60°C for 2 hours. The reactant was then poured into an ice-water, filtered, washed with water to be neutral and dried to yield 99.9g of 2,4-dichloro-5-fluoronitrobenzene (0.476mol) as a slightly yellow crystal, in 95.1% yield, mp 39-42°C.

### b. Preparation of 3-bromo-2,4-dichloro-5-fluoronitrobenzene

96.6g of 2,4-dichloro-5-fluoronitrobenzene (0.46mol) was stirred with 1150ml of glacial acetate acid containing 9.7ml of bromine. Then, a solution consisting of 690ml of concentrated sulfuric acid and 464ml of water was added thereto. To the resultant was added 67.5g of potassium bromate seven times (9.65g each) at 40°C over around 2 hours. The reaction was ceased after 8 hours. The reacting mixture was slowly poured into 3000ml of water containing 46.0g of sodium bisulfite, and kept overnight. The resultant was filtered, and washed with warm water (about 30°C) from three to four times, and dried to give a primrose crystal of 3-bromo-2,4-dichloro-5-fluoronitrobenzene, 122.1g (0.423mol), mp 51-53.5°C, in 91.9% yield.

### c. Preparation of 2,4-dichloro-5-fluoro-3-methoxylnitrobenzene

120g of 3-bromo-2,4-dichloro-5-fluoronitrobenzene (0.415mol), 1.66g of NaOH (0.042mol) and 26.9g of sodium methoxide (0.498mol) in 500ml of methanol were stirred at 60°C for 4 hours. The reactant was extracted with ethyl ether, washed successively with water, dilute hydrochloric acid solution, and water, dried over anhydrous sodium sulfate, and evaporated. The residue was recrystallized from ethanol to give 73.5 g of 2,4-dichloro-5-fluoro-3-methoxynitrobenzene (0.306mol) in 73.8% yield.

### d. Preparation of 2,4-dichloro-5-fluoro-3-methoxyaniline

72.0g of 2,4-dichloro-5-fluoro-3-methoxynitrobenzene (0.30mol) and 2.65g of Pd/C in 500ml of methanol were hydrogenated under 3Mpa (hydrogen pressure) at 60°C for 3 hours. The reactant was cooled to room temperature. The catalyst was filtered out, and the solvent was evaporated to give 58.5g of 2,4-dichloro-5-fluoro-3-methoxyaniline (0.279mol) in 92.9% yield.

### e. 2,4-dichloro-5-fluoro-3-methoxybromobenzene

To 165ml of a mixture of concentrated hydrochloric acid and water (1:1) was added 52.5g of 2,4-dichloro-5-fluoro-3-methoxyaniline (0.25mol). The resultant was stirred to be a slurry, and cooled to 0-5°C. Over 30 minutes, a solution of sodium nitrite (18.2g) in water (50ml) was added dropwise to the slurry. The resulting solution was added slowly to a solution of cuprous bromide (0.299mol) and hydrobromic acid (160ml). The reactant was stirred for 2 hours, and kept overnight. The resulting mixture was wet-distilled and the distilled materials were extracted with toluene. The oil layer was washed with 10% aqueous solution of NaOH, concentric sulfuric acid and water, dried over anhydrous sodium sulfate, and evaporated. The residue was purified by silica gel chromatography eluting with n-hexane-dichloromethane to give 58.1g of 2,4-dichloro-5-fluoro-3-methoxybromobenzene (0.212mol) in 84.9% yield.

### f. Preparation of 2,4-dichloro-5-fluoro-3-methoxyacetylbenzene

0.20mol of 2,4-dichloro-5-fluoro-3-methoxybromobenzene, 0.22mol of powder of magnesium, 0.24mol of cuprous chloride in 240ml of tetrahydrofuran were stirred at ambient temperature to initiate the reaction, and then quickly cooled to -30± 10°C. The reaction was monitored by TLC to be finished. A solution of acetyl chloride (0.30mol) in 50ml of toluene was added dropwise to the resulting mixture. The reaction was carried out at -30±10°C for 8 hours. The temperature of the reaction was then increased to room temperature and stirred for 3 hours. The reactant was poured into a mixture of 170g of crushed ice containing 75ml of 25% aqueous solution of sulfuric acid. The resulting mixture was stirred for 5 minutes. The organic phase was separated off. The aqueous layer was extracted by toluene. The combined organic phases were washed successively with aqueous solution of sodium chloride, aqueous solution of sodium bicarbonate and water, and dried over anhydrous sodium sulfate. The solvent was evaporated under reduced pressure. The residue was recrystallized from ethanol to afford 38.7g of 2,4-dichloro-5-fluoro-3-methoxyacetylbenzene (0.163mol) in 81.6% yield, mp. 36.7-39.4°C.

### Example 6 Preparation of 3-cyano-2,4-dichloro-5-fluoroacetylbenzene

### a. Preparation of 3-cyano-2,4-dichloro-5-fluoronitrobenzene

120g of 3-bromo-2,4-dichrolo-5-fluorobenzene (0.415mol) as prepared in Example 5, 36.9g of anhydrous cuprous cyanide (0.46mol) and 200ml of DMF were stirred under waterless atmosphere and heated to 100°C for 6 hours. TLC monitored the reaction to the completion. The reactant was cooled to room temperature, and then added to a solution of iron trichloride (83g), concentrated hydrochloric acid (5.5ml) and 830ml of water. The resulting mixture was stirred at 50-60°C for 30 minutes. The reactant was extracted with ethyl ether. The organic layer was washed with brine and dried over anhydrous MgSO₄. After the solvent was evaporated off, the residue was purified by silicon gel chromatography eluting with toluene. The crude product was recrystallized from methanol to yield 58.5g of 3-cyano-2,4-dichloro-5-fluoronitrobenzene (0.249mol) in 60.0% yeild.

### b. Preparation of 3-cyano-2,4-dichloro-5-fluoroaniline

27.0g of 3-cyano-2,4-dichloro-5-fluoronitrobenzene (0.247mol), 500ml of ethanol and 2.18g of Pd/C were heated to 60°C and hydrogenated under 3Mpa of hydrogen pressure for 3 hours. The reaction mixture was cooled to room temperature. The catalyst was filtered off. After removing the solvent, 48.0g of 3-cyano-2,4-dichloro-5-fluoroaniline were given in 94.7 yield.

### c. Preparation of 3-cyano-2,4-dichloro-5-fluorobromobenzene

47.2g of 3-cyano-2,4-dichloro-5-fluoroaniline (0.23mol) were added to a solution of 150ml of hydrochloric acid-water (1:1). The resulting mixture was stirred to be a slurry and then cooled to 0-5°C. A solution of sodium nitrite (16.7g, 0.242mol) and water (46ml) was added thereto over 30 minutes. The resulting solution was added to a solution of cuprous bromide (0.275mol) in 147ml of hydrobromic acid. The reaction mixture was stirred for 2 hours and allowed overnight. The resulting mixture was wet-distilled. The distilled fraction was extracted with toluene. The oil layer was washed successively with 10% aqueous solution of NaOH, concentrated sulfuric acid and water, and dried over anhydrous sodium sulfate. After removing the solvent, the residue was crystallized from toluene to give 53.3g of 3-cyano-2,4-dichloro-5-fluorobromobenzene (0.198mol) in 86.2% yield.

### d. Preparation of 3-cynao-2,4-dichloro-5-fluoroacteylbenzene

0.18mol of 3-cyano-2,4-dichloro-5-fluorobromobenzene, powder of magnesium (0.198mol), cuprous chloride (0.216mol) and 216ml of THF were stirred to initiate the reaction at room temperature. The reaction then was quickly cooled to -30±10°C. TLC was used to monitored the reaction to the completion. Then, to the reaction mixture was added 21.2g of acetyl chloride (0.27mol) in 45ml of toluene. The reaction was carried out at -30±10°C for 8 hours. The reaction was then heated to room temperature and stirred for 3 hours. The reactant was then poured into 150g of crushed ice containing 75ml of 25% sulfuric acid solution and stirred for 5 minutes. The organic layer was separated. The aqueous layer was extracted with toluene. The combined organic layers were washed with aqueous solution of NaCl, aqueous solution of NaHCO₃ and water, and dried over anhydrous sodium sulfate. The solvent was evaporated under reduced pressure. The residue was crystallized form ethanol to give 0.148 mol of 3-cynao-2,4-dichloro-5-fluoroacteylbenzene in 82.3% yield, mp. 94.3-96.0°C.

### Example 7 2,4,6-trichloro-3,5-difluoroacetylbenzene

### a. Preparation of 3,5-dichloro-2,4-dichloronitrobenzene

100ml of tetrachloro methane and 30.2g of 2,4-difluoronitobenzene (0.19mol) were introduced into a three-necked flask equipped with a reflux condenser, a thermometer and a stirrer. Chlorine gas was introduced under reflux for about three hours and the reaction was monitored by TLC. After the reaction was finished, terachloromethane was evaporated and the residue was recrystallized from ethyl acetate to give 39.0g of 3,5-dichloro-2,4-dichloronitrobenzene (0.171mol) in 90.0% yield, m.p. 41-44°C.

### b. Preparation of 3,5-dichloro-2,4-difluoroaniline

38.5g of 3,5-difluoro-2,4-difluoronitrobenzene (0.169mol) and 1.49g of Pd/C were introduced into a 100ml autoclave. Hydrogen gas was introduced into the autoclave under 3Mpa of hydrogen pressure at 60°C for three hours. The reactant was cooled to room temperature. The catalyst was filtered off, and the resulting mixture was distilled to give 31.8g of 3,5-dichloro-2,4-difluoroaniline (0.161mol) in 95.1% yield.

### c. Preparation of 2,4-difluoro-1,3,5-trichlorobenzene

31.1g of 3,5-dichloro-2,4-difluoroaniline (0.157mol) was added to 100ml of a solution of concentrated hydrochloric acid and water (1:1) at 70°C. The resulting mixture was stirred to be a slurry and cooled to 0-5°C. To the mixture was added 11.6g of aqueous solution of sodium nitrite (0.165mol) over 30 minutes. The resulting solution was added to a solution of CuCl (0.188mol) and hydrochloric acid (100ml). The reaction mixture was stirred for 2 hours and kept overnight. Fractions obtained by wet-distillation were extracted with toluene. The extract was washed successively with 10% aqueous solution of NaOH, concentrated sulfuric acid and water, dried over sodium sulfate and evaporated. The residue was recrystallized from toluene to give 27.7g of 2,4-difluoro-1,3,5-trichlorobenzene (0.127mol) in 81.1% yield.

### d. Preparation of 2,4,6-trichloro-3,5-difluorobromobenzene

A solution of 186ml of concentrated sulfuric acid and 125ml of water was slowly added to 27g of 2,4-difluoro-1,3,5-trichlorobenzene (0.124mol) and 310ml of glacial acetic acid containing 2.6ml of bromine in a reaction flask under agitation. 18.2g of KBrO₃ was added to the flask in 7 times (2.6g each, around 1 hour) at 45°C under agitation. The reaction was ceased after 8 hours. The reaction materials were poured into 1,000ml of aqueous solution of NaHS03 (12.4g) and kept overnight. The resulting mixture was filtered. The precipitate was washed with warm water 3-4 times, and dried to give 34.1g of 2,4,6-trichloro-3,5-difluorobenzene (0.115mol) in 92.9% yield.

### e. Preparation of 2,4,6-trichloro-3.5-difluoroacetylbenzene

0.1mol of 3,5-difluoro-2,4,6-trichlorobromobenzene, powder of magnesium (0.11mol), cuprous chloride (0.12mol) in 120ml of THF were stirred at room temperature until the reaction mixture appeared black, which showed the reaction had been initiated. The reaction then was quickly cooled to -30±10°C. GLC was used to monitored the reaction to the completion. Then, to the reaction mixture was added dropwise 11.78g of acetyl chloride (0.27mol) in 25ml of toluene. The reaction was carried out at -30±10°C for 8 hours. The reaction was then heated to room temperature and stirred for 3 hours. The reactant was poured into 150g of crushed ice containing 75ml of 25% sulfuric acid solution and stirred for 5 minutes. The organic layer was separated. The aqueous layer was extracted with toluene. The combined organic layers were washed with aqueous solution (45ml) of NaCl, aqueous solution (44ml) of NaHCO₃, water (45ml) and saturated aquoues solution (25ml) of NaCl, and dried over anhydrous sodium sulfate. The solvent was evaporated under reduced pressure. The residue was recrystallized form ethanol to give 0.0819 mol of 3,5-difluoro-2,4,6-trichloroacteylbenzene in 81.9% yield, mp. 73-76°C.

## Claims

1. A process for preparing quinolinecarboxylic acid derivatives having the formula (I): 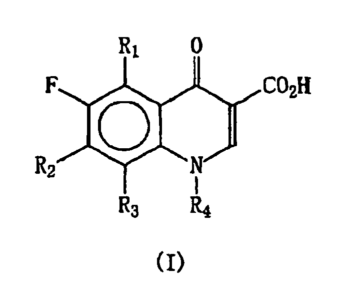 wherein R₁ is H, halogen, or amino,
R₂ is halogen,
R₃ is halogen, C₁₋₄ alkoxyl, or CN,
R₄ is selected from the group consisting of C₃₋₆cycloalkyl, C₁₋₄alkyL C₁₋₄alkoxyC₁₋₄alkyl, and C₁₋₄alkylaminoC₁₋₄alkyl,
comprising the steps of:
i) reacting a compound of the formula (II) 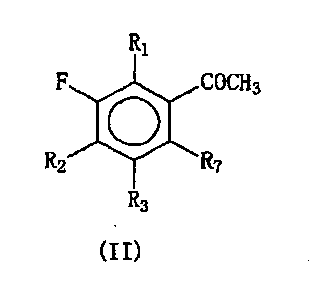 wherein R₁, R₂ and R₃ are defined as above, and R₇ is a halogen, with an alkyl carbonate to obtain a compound of the formula (III), 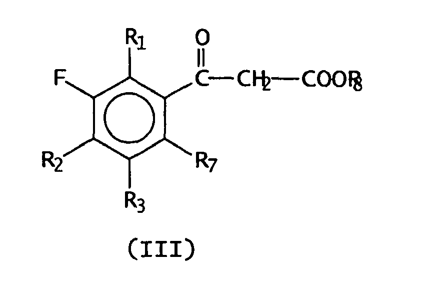 wherein R₈ is methyl or ethyl;
ii) reacting a compound of the formula (III) successively with an alkyl orthoformate and a compound of the formula (IV) R₄NH₂ to obtain a compound of the formula (V), 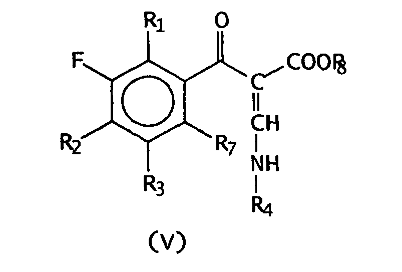
iii) cyclizing the compound of the formula (V) in the presence of a base to form a compound of the formula (VI); and 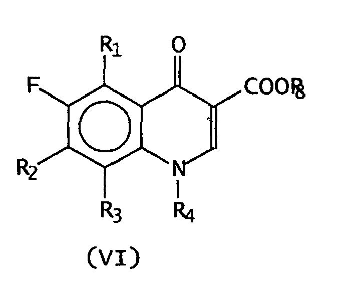
iv) hydrolyzing the compound of the formula (VI) to obtain a compound of the formula (I).

2. A process according to claim 1, wherein R₁ is selected form the group consisting of H, F, Cl, and NH₂; R₂ is Cl or F; R₃ is selected from the group consisting of F, Cl, CN, and C₁₋4alkoxy, and R₄ is a group selected from C₃₋₆cycloalkyl.

3. A process according to claim 2, where R₃ is F, CN or OCH₃; and R₄ is a group selected from C₃₋₆cylcoalkyl.

4. A process according to claim 3, wherein R₁ is H, F, or NH₂; R₂ is F; and R₄ is cyclopropyl or cyclohexyl.

5. A process according to claim 1 further comprising in step iv) converting a compound of the formula (I) in which R₁ is halogen to that in which R₁ is amino.

6. A compound of the formula (II): 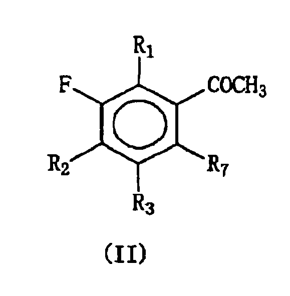 wherein, R₁ is H, or Cl; R₂ is Cl; R₃ is C₁₋₄ alkoxyl or CN; and R₇ is Cl.

7. A compound according to claim 6, wherein R₃ is OCH₃ or CN.

8. A compound according to claim 7, wherein R₁ is H.

## Patentansprüche

1. Verfahrung zur Herstellung von Chinolincarbonsäurederivaten der Formel (1): 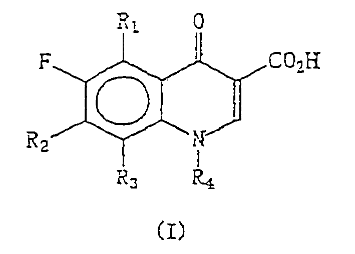 in der
R₁ H, Halogen oder Amino,
R₂ Halogen,
R₃ Halogen, C₁₋₄-Alkoxyl oder CN bedeuten,
R₄ aus der Gruppe bestehend aus C₃₋₆-Cycloalkyl, C₁₋₄-alkyl, C₁₋₄-Alkoxy-C₁₋₄-alkyl, und C₁₋₄-Alkylamino-C₁₋₄-alkyl ausgewählt ist,
enthaltend die Schritte:
i) man setzt eine Verbindung der Formel (II): 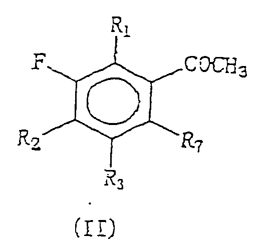 in der R₁, R₂ und R₃ die oben beschriebene Bedeutungen besitzen und R₇ Halogen ist, mit einem Alkylcarbonat um zur Erzielung einer Verbindung der Formel (111):, 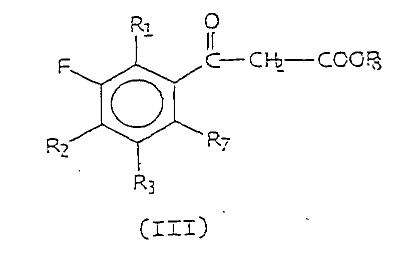 in der R₈ Methyl oder Ethyl ist;
ii) man setzt eine Verbindung der Formel (III) nacheinander mit einem Alkylorthoformiat und einer Verbindung der Formel (IV) R₄NH₂ zu einer Verbindung der Formel (V) um, 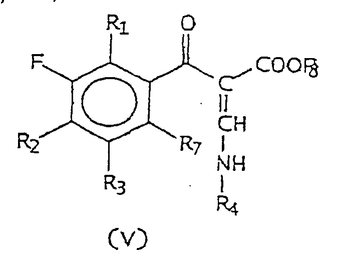
iii) man zyklisiert die Verbindung der Formel (V) in Anwesenheit einer Base zur Bildung einer Verbindung der Formel (V1); und 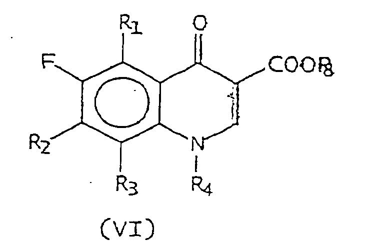
iv) man hydrolisiert die Verbindung der Formel (VI) zur Erzielung einer Verbindung der Formel (I).

2. Verfahren nach Anspruch 1, wobei R₁ aus der Gruppe H, F, Cl und NH₂ ausgewählt ist; R₂ Cl oder F ist; R₃ aus der Gruppe F, Cl, CN und C₁₋₄-Alkoxy ausgewählt ist; und R₄ eine Gruppe ausgewählt aus C₃₋₆₋Cycloalkyl ist.

3. Verfahren nach Anspruch 2, wobei R₃ F, CN oder OCH₃ ist; und R4 eine Gruppe ausgewählt aus C₃₋₆-Cycloalkyl ist.

4. Verfahren nach Anspruch 3, wobei R₁ H, F oder NH₂; R₂ F; und R₄ Cyclopropyl oder Cyclohexyl ist,

5. Verfahren nach Anspruch 1, weiterhin enthaltend in Stufe iv) die Umwandlung einer Verbindung der Formel (1), in der R₁ Halogen ist, in eine solche, in der R₁ Amino ist.

6. Verbindung der Formel (II): 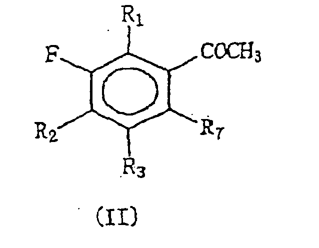 in der R₁ H oder Cl; R₂ Cl; R₃ C₁₋₄-Alkoxyl oder CN; und R₇ Cl ist.

7. Verbindung nach Anspruch 6, wobei R₃ OCH₃ oder CN ist.

8. Verbindung nach Anspruch 7, wobei R₁ H ist.

## Revendications

1. Procédé pour préparer les dérivés d'acide quinolinecarboxiliques ayant la formule (I) : 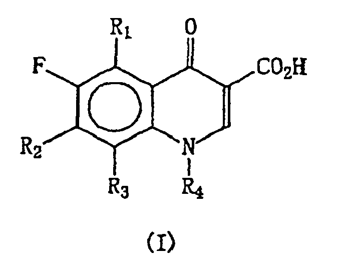 Où R₁ est H, halogène, ou amino,
R₂ est halogène
R₃ est halogène, C₁₋₄alkoxyle, ou CN
R₄ est sélectionné parmi le groupe consistant en : C₃₋₆cycloalkyle, C₁₋₄alkyle, C₁₋₄alkoxy C₁₋₄alkyle, et C₁₋₄alkylaminoC₁₋₄alkyle,
Comprenant les étapes de :
i) la réaction d'un composé de la formule (II) 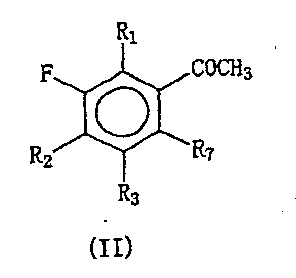 où R₁, R₂ et R₃ sont définis comme ci-dessus, et R₇ est un halogène, avec un alkyle carbonate pour obtenir un composé de formule (III) : 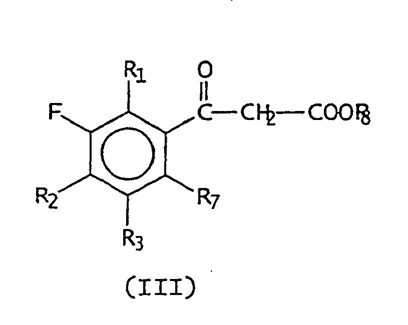 où R₈ est méthyle ou éthyle ;
ii) la réaction d'un composé de la formule (III) successivement avec un alkyle orthoformate et un composé de la formule (IV) R₄NH₂ un composé de la formule (V), 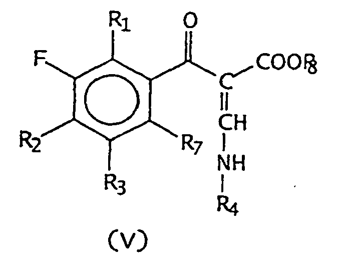
iii) la cyclisation du composé de la formule (V) en présence d'une base pour former le composé de la formule (VI) ; et 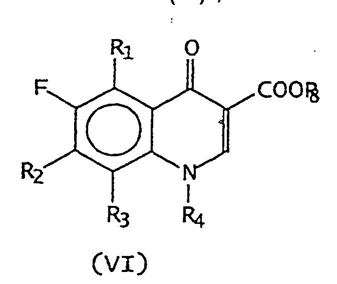
iv) l'hydrolyse du composé selon la formule (VI) pour obtenir un composé de la formule (I).

2. Procédé selon la revendication 1, où R₁ est sélectionné parmi le groupe consistant en H, F, CI, et NH₂ ; R₂ est Cl ou F ; R₃ est sélectionné parmi le groupe consistant en F, CI, CN, et C₁₋₄alkoxyle ; et R₄ est un groupe sélectionné parmi C₃₋₆cycloalkyle.

3. Procédé selon la revendication 2, où R₃ est F, CN ou OCH₃ ; et R₄ est un groupe sélectionné parmi C₃₋₆cycloalkyle.

4. Procédé selon la revendication 3, où R₁ est H, F, ou NH₂ ; R₂ est F; et R₄ est cyclopropyle ou cyclohexyle.

5. Procédé selon la revendication 1 comprenant aussi l'étape (iv) convertissant un composé de formule (I) pour lequel R₁ est un halogène en R₁ est amino.

6. Composé selon la formule (II) : 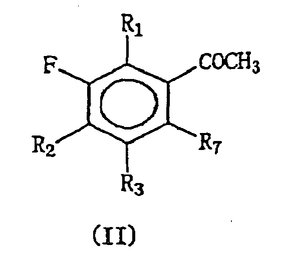 où R₁ est H, ou CI ; R₃ est C₁₋₄alkoxyle ou CN ; et R₇ est CI.

7. Composé selon la revendication 6, où R₃ est OCH₃ ou CN.

8. Composé selon la revendication 7, où R₁ est H.
